# EUROPÄISCHE PATENTSCHRIFT

(11) **EP 3 581 155 B1**
(45) Veröffentlichungstag und Bekanntmachung des Hinweises auf die Patenterteilung: **07.09.2022**
(21) Anmeldenummer: 19177822.4
(22) Anmeldetag: 03.06.2019
(51) Int. Cl.: A61F 2/82, A61L 31/08, C25D 11/00, A61B 90/00

(54) **FUNKTIONSMARKERELEMENT UND VERFAHREN ZU DESSEN HERSTELLUNG**
FUNCTIONAL MARKING ELEMENT AND METHOD OF MANUFACTURING SAME
ÉLÉMENT MARQUEUR FONCTIONNEL ET SON PROCÉDÉ DE FABRICATION

(30) Priorität: 11.06.2018 DE 102018113810
(43) Veröffentlichungstag der Anmeldung: 18.12.2019
(73) Patentinhaber: Cortronik GmbH, 18119 Rostock-Warnemünde (DE)
(72) Erfinder: Bayer, Ullrich, 18209 Bad Doberan (DE); Rüter, Björn, 18211 Ostseebad Nienhagen (DE); Knop, Uwe, 18055 Rostock (DE); Berngruber, Gesa, 18059 Papendorf (DE); Grabow, Niels, 18055 Rostock (DE); Illner, Sabine, 18059 Rostock (DE); Eickner, Thomas, 18059 Rostock (DE); Schmitz, Klaus-Peter, 18119 Rostock (DE)
(74) Vertreter: Biotronik Corporate Services SE

(56) Entgegenhaltungen:
- EP-A1- 3 348 239
- EP-A2- 2 399 619
- EP-B1- 2 399 619
- DE-B3-102013 020 689
- US-A1- 2018 042 693

## Beschreibung

Die vorliegende Erfindung bezieht sich auf ein Markerelement für ein Implantat und ein Gerüst für ein Implantat mit einem Markerelement sowie ein Verfahren zur Herstellung eines solchen Markerelements und eines solchen Gerüsts.

Die vorliegende Erfindung betrifft außerdem ein Implantat, insbesondere eine intraluminale Endoprothese, mit einem Gerüst (Scaffold) und mit einem an dem Gerüst befestigten Markerelement, das zumindest in einem Teil seines Volumens eine verglichen mit dem Material des Gerüsts verschiedene Materialzusammensetzung aufweist, die vorzugsweise radioopakes und/oder röntgenopakes Material umfasst, sowie ein Verfahren zur Herstellung eines solchen Implantats.

Die Implantate sind endovaskuläre Prothesen (Endoprothesen, Stents) oder andere Implantate, die zur Behandlung von Stenosen (Gefäßverengungen) eingesetzt werden können. Sie weisen meist ein Gerüst in Form eines hohlzylinder- oder röhrenförmigen Grundgitters auf, das an beiden Längsenden der Röhren offen ist. Ein solches Gerüst weist meist eine Vielzahl von miteinander verbundenen Streben (Struts) auf, die das Grundgitter ausbilden. Das röhrenförmige Grundgitter einer derartigen Endoprothese wird in das zu behandelnde Gefäß eingesetzt und dient dazu, das Gefäß zu stützen. Weitere Gerüstformen sind ebenfalls möglich. Ferner betrifft die vorliegende Erfindung Implantate, welche im Bereich der Orthopädie verwendet werden können, z.B. für den Schädelbereich, und insbesondere Implantate, die auf Grund ihrer geringen Größe und Wanddicke eine geringe Röntgensichtbarkeit aufweisen. Die Erfindung kann ebenfalls für Stents im neurovaskulären Bereich zur Anwendung kommen. Hier kommt es darauf an, die hirnversorgenden Blutgefäße mit absorbierbaren Mg-Stents offen zu halten. Diese Systeme kommen auf dem Gebiet der Verhinderung akuter ischämischer Schlaganfälle zum Einsatz.

Stents oder andere Implantate weisen in ihrem Gerüst häufig metallische Materialien auf. Hierbei können die metallischen Materialien einen biodegradierbaren Werkstoff bilden, wobei auch polymere biodegradierbare Materialien enthalten sein können.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile des Gerüsts bzw. des Implantats führen. Häufig wird auch der Begriff Biokorrosion bei für den biologischen Degradationsprozess bei Implantaten aus Metallen verwendet. Der Begriff Bioresorption umfasst die anschließende Resorption der Abbauprodukte durch den lebenden Organismus. Das Ziel der Verwendung biodegradierbarer Implantate besteht darin, dass sie zu einem Zeitpunkt, wenn sie beispielsweise in Bezug auf ihre Stützwirkung nicht mehr benötigt werden, vom Organismus abgebaut sind und demzufolge nicht länger als nötig als Fremdkörper im Organismus vorhanden sind.

Für das Gerüst biodegradierbarer Implantate geeignete Werkstoffe (Grundmaterial) können aus einem Material oder mehreren Materialien bestehen. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglycolsäure (PGA), Poly-D,L-Lactid-co-glycolid (PDLLA-co-GA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen, Zink und/oder Wolfram.

Die Ermittlung der Position eines Stents oder anderer Implantate geschieht häufig mittels bildgebender Verfahren, beispielsweise mittels einer Röntgenstrahleinrichtung. Auf Grund der kleinen Ordnungszahl und der geringen Dichte des biodegradierbaren Materials, z.B. Magnesium und seiner Legierungen, ist die Röntgensichtbarkeit der daraus gefertigten medizinischen Implantate sehr gering. Um diesen Nachteil zu beheben, ist es bekannt, medizinische Vorrichtungen mit Markerelementen zu versehen, die zumindest in einem Teil ihres Volumens eine verglichen mit dem Material des Gerüsts verschiedene Materialzusammensetzung aufweisen. Diese sogenannten (Röntgen-)Marker oder Markerelemente enthalten insbesondere ein Material, das die Röntgenstrahlen und/oder andere elektromagnetische Strahlen stärker absorbiert (im Folgenden als röntgenopakes oder radioopakes Material bezeichnet) als das Material des Gerüsts bzw. die Körperumgebung des Patienten und hierdurch relativ zu seiner Umgebung sichtbar wird. Basierend auf der ermittelten Lage der meist mehreren Markerelemente an dem Gerüst lässt sich die Lage und Winkelposition des Implantats in Bezug auf die umgebenden Organe bestimmen. Bei Verwendung eines biodegradierbaren Gerüsts wird aus Gründen der ausreichenden Röntgensichtbarkeit häufig ein nicht resorbierbares, röntgenopakes oder radioopakes Material (z.B. Ta, Au, W) eingesetzt.

Ein solches Markerelement wird häufig aus einem Halbzeug bestehend aus dem Material des Markerelements ab- oder ausgeschnitten und derart in ein Implantat integriert, dass es in eine entsprechende Öffnung (Eyelet), die hierfür am Gerüst des Implantats vorgesehen ist (z.B. an beiden Enden in axialer Richtung des Implantats), eingeklebt wird. Derartige Markerelemente und Implantate sind beispielsweise aus den Druckschriften EP 2 399 619 B1 und EP 3 165 238 A1 bekannt.

US 2018/042693 offenbart ein medizinisches Implantat mit mindestens einer Öffnung für einen Röntgenmarker. Der Röntgenmarker weist eine Beschichtung zur Vermeidung von Kontaktkorrosion und dem Implantat auf.

EP 3 348 239 ist Stand der Technik unter Artikel 54(3) EPÜ und offenbart ein Verfahren zur Produktion von Röntgenmarkern. Dabei wird eine vorbestimmte Sollbruchstelle durchtrennt, um den Röntgenmarker aus einer Materiallage heraus zu lösen, nachdem der Marker und die Materiallage einer plasmaelektrolytischen Behandlung ausgesetzt war.

Es besteht die technische Anforderung an Implantate mit integrierten Markerelementen, dass die Markerelemente über einen langen Zeitraum mit einer ausreichenden Adhäsionskraft im Gerüstverbund verbleiben. Dies geschieht bisher ausschließlich mit technologischen Mitteln, die entweder stoff-, form oder kraftschlüssig in Bezug auf das Gerüst bzw. die Öffnung des Gerüsts sein können, in die das Markerelement eingebracht ist. Bei einer nicht ausreichenden Anbindung des Markerelements an das Gerüst des Implantats kann sich durch einen metallischen Kontakt zwischen Markerelement und Gerüstmaterial ein unerwünschtes Lokalelement ausbilden oder Kontaktkorrosion verursacht werden. Dies würde zu einer frühzeitigen Abtrennung des Markerelements von dem Gerüst des Implantats führen, was eine nicht gewünschte Fragmentbildung und Embolisation begünstigen würde. Zudem soll generell verhindert werden, dass das Material des Markerelements einen Einfluss auf die Degradation des Gerüsts hat.

Die Aufgabe der vorliegenden Erfindung besteht somit darin, ein Implantat bzw. ein Gerüst für ein Implantat zu schaffen, bei dem eine Lokalelementbildung sowie Korrosion zumindest reduziert wird. Entsprechend sollte ein Markerelement geschaffen werden, das nach der Integration in das Implantat kein Lokalelement bildet und Korrosion vermeidet. Weiter sollen kostengünstige, einfache und einfach zu automatisierende Verfahren zur Herstellung eines derartigen Markerelements, eines Gerüsts mit einem solchen integrierten Markerelement oder eines Implants mit einem derartigen Gerüst angegeben werden.

Die Aufgabe wird gelöst durch das in Anspruch 1 angegebenen Verfahren zur Herstellung eines Markerelements , sowie durch das in Anspruch 11 spezifizierte scheibenförmige Markerelement für ein Implantat.

Das erfindungsgemäße Verfahren zur Herstellung eines Markerelements für ein Implantat aus einem plattenförmigen oder hohlkörperförmigen Halbzeug bestehend aus dem Material des Markerelements weist insbesondere die folgenden Schritte auf:
- Ausschneiden mindestens eines Abschnitts aus dem Halbzeug derart, dass der Abschnitt über mindestens einen Steg mit dem übrigen Material des Halbzeugs verbunden ist,
- während der Abschnitt weiter über den mindestens einen Steg mit dem Halbzeug verbunden ist:
   - Abdecken einer ersten Oberfläche des Abschnitts und
   - anschließende plasmaelektrolytische Behandlung mindestens einer zweiten Oberfläche des Abschnitts, wobei die mindestens eine zweite Oberfläche von der ersten Oberfläche verschieden ist, und der Seitenfläche des Markerelements, die quer zur zweiten Oberfläche verläuft, wobei durch die plasmaelektrolytische Behandlung eine poröse Schicht zumindest an der zweiten Oberfläche des Abschnitts und zumindest teilweise an der Seitenfläche ausgebildet wird, sowie
- Abtrennen des Abschnitts mittels Durchtrennen des mindestens einen Stegs, wobei
der abgetrennte Abschnitt des Halbzeugs das Markerelement bildet.

Das erfindungsgemäße Verfahren bewirkt eine topografische Strukturierung der nach der Integration in das Gerüst eines Implantats als abluminale Seite des Markerelements dienenden zweiten Oberfläche des Markerelements mit der Option der Beschleunigung des Einwachsens des Markerelements in das Gewebe.

Auf der zweiten Oberfläche und teilweise auf mindestens einer Seitenfläche des Markerelements, die quer zur zweiten Oberfläche verläuft, wird durch die plasmaelektrolytische Oxidation eine hohe Oberflächenporosität erzeugt, die dazu dienen kann, einen hohen Benetzungsgrad für nachfolgend aufzubringende Beschichtungen zu erzielen.

Das plattenförmige oder hohlkörperförmige Halbzeug wird beispielsweise durch eine Platte, einen Hohlzylinder (Rohr) oder ein dreiseitiges, vierseitiges oder mehr als vierseitiges, vorzugsweise gerades Hohlprisma, das an beiden Enden in Richtung einer Längsachse offen ist, gebildet.

Das Halbzeug und entsprechend das aus dem Halbzeug hergestellte Markerelement besteht aus einem Metall oder einer Metalllegierung enthaltend mindestens ein Metall der Gruppe mit den Elementen Wolfram, Tantal, Gold und Platin. Besonders bevorzugt besteht das Halbzeug und entsprechend das Markerelement aus Tantal oder einer Tantallegierung. Die angegebenen Materialen des Markerelements besitzen gute Röntgenabsorptionseigenschaften und lassen sich durch die plasmaelektrolytische Oxidation an ihrer Oberfläche sehr einfach mit einer dichten, passivierenden und isolierenden (d.h. elektrisch nicht leitenden) Oxidschicht versehen. Sie erlauben hierdurch eine Gestaltung eines Markerelements mit minimaler Größe. Vorzugsweise wird das Halbzeug durch Ziehen aus dem jeweiligen Material hergestellt.

Das Ausschneiden des mindestens einen Abschnitts aus dem Halbzeug, vorzugsweise einer Vielzahl von Abschnitten nebeneinander angeordnet entlang der gesamten Breite der Platte oder des gesamten oder teilweisen Umfangs des Hohlzylinders oder Hohlprismas, erfolgt derart, dass jeder Abschnitt noch über mindestens einen Steg mit dem übrigen Material des Halbzeugs verbunden ist. Vorzugsweise ist jeder Abschnitt über mindestens zwei Stege, die entlang des Umfangs des Abschnitts gegenüber liegen, mit dem übrigen Material des Halbzeugs verbunden. Jeder Steg bildet eine Sollbruchstelle für das Abtrennen des jeweiligen Abschnitts von dem Halbzeug. Das Ausschneiden jedes Abschnitts aus dem Halbzeug, vorzugsweise mittels Laser, erfolgt derart, dass der jeweilige Abschnitt entlang seines Umfangs (bis auf den/die Steg(e)) vom Halbzeug freigestellt ist, d.h. dass ein umlaufender, durchgehender Einschnitt das Material des jeweiligen Abschnitts (bis auf den/die Steg(e)) von dem übrigen Material des Halbzeugs trennt. Jeder Abschnitt besitzt (abgesehen von dem/den Steg(en)) bereits die Form des hieraus hergestellten Markerelements, beispielsweise eine Scheibenform. Die Scheibe kann eine kreisförmige, elliptische, dreieckige, viereckige oder sonstige vieleckige oder abgerundete Grundform aufweisen, die jeweils durch die Grund- und Deckfläche gebildet wird. Insbesondere weist jeder Abschnitt eine erste Oberfläche, die die Grundfläche des Markerelements ausbildet, eine der ersten Oberfläche gegenüber liegenden zweite Oberfläche, die die Deckfläche des Marker-elements ausbildet, sowie mindestens eine Seitenfläche auf, die die erste und die zweite Oberfläche miteinander verbindet. Die Höhe der Seitenfläche entspricht der Dicke des Abschnitts bzw. des hieraus hergestellten Markerelements, wobei bei der Bestimmung der Dicke des Markerelements die plasmaelektrolytisch erzeugte Beschichtung und ggf. weitere Beschichtungen berücksichtigt werden müssen. Ferner entspricht die Höhe der Seitenfläche auch der Dicke der Halbzeug-Platte bzw. der Wandstärke des Hohlzylinders bzw. des Hohlprismas. Die mindestens eine Seitenfläche entspricht gewissermaßen der Schneidkante des ausgeschnittenen Abschnitts.

Durch die plasmaelektrolytische Behandlung entsteht eine poröse, dielektrische Oberfläche an den nicht abgedeckten Flächen, insbesondere der zweiten Oberfläche und ggf. der mindestens einen Seitenfläche, die optional in zusätzlichen Tauchschritten weiter modifiziert werden kann (siehe unten). Für ein Halbzeug bestehend aus Tantal oder einer Tantallegierung erhält die bereits vorhandene, natürliche kristalline Oxidschicht durch diesen Prozess eine amorphe Oxidschicht mit einer Schichtdicke zwischen 0,3 µm und 10 µm, vorzugsweise zwischen 0,5 µm und 4 µm. Tantalmarker besitzen am Ende der plasmaelektrolytischen Behandlung eine Porenstruktur mit einem mittleren Porendurchmesser zwischen 0,1 µm und 2 µm, die mittlere Porentiefe ist etwas kleiner als die Schichtdicke. Hierbei lässt sich der Porendurchmesser durch Rastermikroskopaufnahmen (REM) oder konfokales Laserscanning (CLSM) bestimmen. Die Porentiefe wird im metallografischen Querschliff ebenfalls mit vorgenannten Methoden bestimmt.

Die Abdeckung der ersten Oberfläche des Abschnitts kann bei Verwendung eines Halbzeugs in Form eines Hohlzylinders oder Hohlprismas beispielsweise durch das Einbringen eines langgestreckten (etwa zylinderförmigen) polymeren Ballons oder einen thermisch unterkühlten Schlauch aus z.B. Silikon oder einen durchmesserveränderlichen Schlauch mit separatem Innendorn in das innere Volumen des Hohlzylinders oder Hohlprismas erfolgen. Mittels Druckbeaufschlagung drückt sich der Ballon an die Innenfläche des Halbzeugs und dichtet somit die innere Oberfläche (einschließlich der ersten (inneren) Oberfläche des mindestens einen ausgeschnittenen Abschnitts für das Markerelement) gegen nachfolgend wirkende äußere Medienzutritte ab. An der abgedeckten ersten (inneren) Oberfläche des mindestens einen ausgeschnittenen Abschnitts des Halbzeugs erfolgt somit keine Veränderung der Oberfläche durch die plasmaelektrolytische Behandlung.

Für die plasmaelektrolytische Behandlung wird das Halbzeug elektrisch kontaktiert und mit Hilfe des plasmaelektrolytischen Prozesses, der in einer spezifischen Säuremischung bei hohen Badspannungen durchgeführt wird, beschichtet. Aufgrund der Abdeckung der ersten (inneren) Oberfläche des ausgeschnittenen Abschnitts erfolgt dort keine Beschichtung, sondern nur an den frei liegenden Oberflächen, d.h. an der nicht abgedeckten zweiten Oberfläche und gegebenenfalls an mindestens einer Seitenfläche des mindestens einen ausgeschnittenen Abschnitts. Die zweite Oberfläche liegt der ersten Oberfläche gegenüber und ist vorzugsweise parallel zu dieser.

Bei der plasmaelektrolytischen Behandlung (Beschichtung) wird an das Halbzeug mittels anodischer Kontaktierung eine gepulste Spannung angelegt. Über den mindestens einen Steg ist auch der mindestens eine ausgeschnittene Abschnitt in elektrisch leitendem Kontakt mit der Spannungsquelle. Die Amplitude der Spannung übersteigt in einem Teil des Behandlungszeitraums eine für das Material des Grundköpers charakteristische Badspannung und steigt vorzugsweise im Verlauf der Behandlung an. Vorteilhafterweise wird eine Spannung zwischen 450 V und 1.000 V, bevorzugt zwischen 500 V und 600 V angelegt. Der Kontaktierungswerkstoff besteht aus einem geeigneten säurebeständigen Metall beispielsweise aus einem Titan-Draht. Nun wird das Halbzeug in eine säurehaltige Lösung eingetaucht. Nach Anlegen einer gepulsten, stetig steigenden Badspannung, die sich durch lange Pulspausen (Pulse-Off) auszeichnet, kommt es zur Oxidation des Werkstoffs des Halbzeugs mit dem mindestens einen ausgeschnittenen Abschnitt. Dabei sind die Pulspausen (Pulse-Off) mindestens genauso lang wie die Pulsdauer (Pulse-On) bevorzugt aber eineinhalb Mal so lang. In einer Ausgestaltung der Erfindung werden zweckmäßigerweise Pulsdauern in Bereich zwischen 30 µs und 600 µs, bevorzugt zwischen 100 µs und 300 µs, besonders bevorzugt 200 µs, und Pulspausen im Bereich von 175 µs bis 600 µs, bevorzugt zwischen 400 µs und 550 µs, besonders bevorzugt 450 µs.

Vorzugsweise wird die plasmaelektrolytische Behandlung bei einer hohen maximalen anliegenden Spannung, vorzugsweise bei einer maximalen anliegenden Spannung von über 180 V mit einer gepulsten Spannungsquelle durchgeführt. Durch die hohe Spannung wird die Porosität der Oxidschicht bewirkt.

Bevorzugt werden spezifischen Säuremischungen wie beispielsweise konzentrierte Phosphorsäure (85%) oder konzentrierte Phosphorsäure (85%) mit konzentrierter Schwefelsäure (bevorzugt im Verhältnis: 90:10, v/v). Als Spannungsprofil ist ein Rechteck oder Trapezprofil zweckmäßig.

Pulsparameter:
- die Pulse-On und Pulse-Off Zeiten korrelieren miteinander
- zwischen den folgenden Eckpunkten (jeweils Pulse-On zu Pulse-Off Zeit) kann ein Prozessfenster aufgespannt werden:
   - 30 µs zu 175 µs, 30 µs zu 600 µs, 600 µs zu 500 µs, 600 µs zu 600 µs
- die optimalen Pulsparameter liegen bei 200 µs Pulse-On zu 450 µs Pulse-Off

Eine derartige plasmaelektrolytische Behandlung wird bevorzugt für die Dauer von 1 bis 3 min durchgeführt.

Vorzugsweise wird das Halbzeug nach der plasmaelektrolytischen Behandlung in VE-Wasser (vollentsalztes Wasser) gespült.

Das Abtrennen des mindestens einen Abschnitts von dem Halbzeug erfolgt nach der plasmaelektrolytischen Behandlung mittels Durchschneiden (z.B. mittels Laser) oder Durchbrechen des mindestens einen Stegs, mit dem der jeweilige Abschnitt mit dem Halbzeug verbunden ist. Gegebenenfalls wird vor dem Abtrennen des mindestens einen Abschnitts die Abdeckung (z.B. der Ballon) von dem Abschnitt bzw. dem Halbzeug entfernt. Das so entstandene Markerelement kann dann in eine Öffnung (Eyelet) des Gitters eingesetzt und mit diesem verklebt werden. Gegebenenfalls finden vor dem Abtrennen des mindestens einen Abschnitts weitere Behandlungen und/oder Beschichtungen des Abschnitts statt (siehe unten).

Der Vorteil der mittels plasmaelektrolytischer Behandlung auf der zweiten Oberfläche und gegebenenfalls mindestens einer Seitenfläche (der Schneidkante) hergestellten verfahrensbedingt porösen Schicht besteht insbesondere in der oxidischen Grundstruktur, die zu dielektrischen d.h. isolierenden Oberflächeneigenschaften führt und damit nach der Integration in das Gerüst eines Implantats einen Metall-/ Metallkontakt zur Öffnung (Eyelet) und somit dem Gerüst verhindert. Die oxidische Grundstruktur ist zudem mechanisch äußerst robust und lässt sich ohne Schichtbeschädigung plastisch verformen. Dies eröffnet die Möglichkeit einer Automatisierung der Prozesse zur Einbringung der Marker-elemente in das Gerüst (Montage) ohne die Gefahr einer Partikelgenerierung. Mit anderen Worten begünstigt die Schaffung einer robusten, widerstandsfähigen porösen Schicht insbesondere an der mindestens einen Seitenfläche durch das erfindungsgemäße Verfahren ein mechanisches Greifen bei einer automatisierten Montage der Markerelemente in das Gerüst.

In einer Weiterbildung der Erfindung wird das Halbzeug im Bereich des mindestens einen ausgeschnittenen Abschnitts vor dem Abdecken der ersten Oberfläche des Abschnitts und vor der plasmaelektrolytischen Behandlung einer Beizbehandlung mit heißer Kalilauge oder einer anderweitigen flusssäurehaltigen Säuremischung, beispielsweise mittels einer Lösung aus HNO₃ und HF, unterzogen. Hierfür wird das Halbzeug und mit diesem der mindestens eine ausgeschnittene Abschnitt in die Säure getaucht. Hierbei werden (Laser-)Schneidgrate entfernt und Modifizierungen an allen Oberflächen des Abschnitts vorgenommen.

Da die erste Oberfläche des ausgeschnittenen Abschnitts bzw. die Grundfläche des Markerelements während der plasmaelektrolytischen Behandlung abgedichtet ist, wird diese Oberfläche durch die plasmaelektrolytische Behandlung nicht verändert. Sie weist daher bei diesem Ausführungsbeispiel nach der Fertigstellung des Markerelements die durch die Beizbehandlung entstandene Struktur auf. Das Markerelement wird bei der Integration in das Gerüst bzw. das Implantat so angeordnet, dass die erste Oberfläche bzw. die Grundfläche des Markerelements auf der luminalen Seite liegt. Die daraus resultierende luminale Markerelementinnenoberfläche besteht in der 0° zur Gerüstachse orientierten, durch den Beizvorgang hervorgerufenen riefenartigen (rillenartigen) Struktur, die durch in eine Vorzugsrichtung orientierte Körner des Markerelementvolumens hervorgerufen und durch das Ziehen des Halbzeugs verursacht wird. Diese riefenartige Struktur wirkt einer Adhäsion der Blutplättchen entgegen und senkt somit das Thromboserisiko, vorzugsweise auch in einer Zeit nach der Auflösung einer hierauf optional angeordneten PLLA- Schicht.

Anschließend kann das Halbzeug mit dem mindestens einen ausgeschnittenen Abschnitt mittels VE-Wasser gespült werden.

In einem weiteren Ausführungsbeispiel wird das Halbzeug im Bereich des mindestens einen ausgeschnittenen Abschnitts nach der plasmaelektrolytischen Behandlung optional zuerst in eine Lösung getaucht und hierdurch beschichtet, wobei die Lösung einen Crosslinker und mindestens ein Material aus der Gruppe enthaltend Proteine, weitere Wachstumsfaktoren, Enzyme, Antikörper und Peptide aufweist.

Bei diesem Ausführungsbeispiel erfolgt eine zusätzliche Modifikation der Markeroberfläche mit Adhäsionsmolekülen. Vorzugsweise wird das noch abgedeckte, z.B. mit der Ballonstruktur abgedichtete Halbzeug nach der plasmaelektrolytischen Behandlung mit bifunktionellen Crosslinkern und nachfolgend mit Proteinen oder Peptiden beschichtet. Diese Beschichtungsvorgänge erfolgen durch Tauchen des Halbzeugs in eine entsprechende Lösung mit diesen Verbindungen. Die Proteine (z.B. VEGF - vascular endothelial growth factor) und Peptide (z.B. RGD-Peptide (Haftpeptide)) haben die Aufgabe, das Markerelement am Implantationsort zu halten und ihm zu einem schnellen Einwachsen zu verhelfen. Eine unbeabsichtigte Freisetzung des Markerelements nach Degradation des Gerüsts und damit ein Transport des freien Markerelements durch das Gefäßsystem kann so verhindert werden.

Selbst nach Beschichtung des Gerüsts inklusive der Markerelemente mit einer Polymerwirkstoffbeschichtung ist es möglich, dass die Proteine oder Peptide wirksam bleiben und erst nach der Degradation der Schicht zu einer zusätzlichen Fixierung im Gewebe führen. In diesem Fall stellt die Modifikation mit Adhäsionsmolekülen einen Schutzmechanismus dar, der die langfristige Sicherheit des Gerüsts erhöht.

Die Methodik der Protein/Peptidbeschichtung ergibt sich aus zwei Teilschritten, wobei das Halbzeug, beispielsweise der Tantalhohlzylinder, im Bereich des mindestens einen ausgeschnittenen Abschnitts, vorzugsweise mit dem noch im Inneren angeordneten Ballon, nacheinander in die einzelnen Beschichtungslösungen a), b) getaucht wird, nämlich
a) eine Mischung eines Crosslinkers, mit Initiator und Starter in einem geeigneten Lösemittel.

Als Crosslinker können Glutardialdehyd, 1-Ethyl-3-(3-dimethylaminopropyl)carbodiimid/ N-Hydroxysuccinimid (EDC/NHS), Aminopropyltriethoxysilan (APTES) oder andere verwendet werden. Hierbei kann die Reaktion auf der zweiten Oberfläche des ausgeschnittenen Abschnitts auch thermisch aktiviert werden.
b) Eine Mischung der Proteine, wie VEGF, weitere Wachstumsfaktoren, Enzyme oder Antikörper oder Peptide, wie Adhäsionspeptide (RGD-Peptide), die allesamt eine Affinität zu den Zellen der Gefäßwand aufweisen.

Diese binden kovalent an die aktivierte Markerelementoberfläche bzw. an die dort bereits vorhandenen Crosslinker. Hierbei kann die Reaktion auf der Markerelementoberfläche auch thermisch aktiviert werden.

Die Art der Beschichtung wird so gewählt, dass die Beschichtungslösung die Markerelementoberfläche und deren Porenstruktur benetzt. In einem Ausführungsbeispiel werden durch Anlegen eines Unterdrucks (z.B. in einem Exsikkator) die Poren der porösen Schicht mit der Protein-/Peptidbeschichtungslösung gefüllt. Danach können entweder Spül- und/oder Trocknungsvorgänge (z.B. mittels VE-Wasser) oder (nach dem Abtrennen von dem Halbzeug) eine sofortige Verklebung in einer Öffnung (Eyelet) des Gerüsts erfolgen.

In einer Weiterbildung der Erfindung werden nach der plasmaelektrolytischen Behandlung eine elutionsfähige organische Verbindung und/oder eine anorganische Verbindung in der Porenstruktur der porösen Schicht immobilisiert.

Als anorganische Verbindungen werden beispielsweise Ca-, K- und Mg-Verbindungen immobilisiert. Dabei kann es sich um physiologisch vorkommende Salzverbindungen wie Phosphate, Chloride oder Carbonate handeln. Da die poröse Schicht auf der zweiten Oberfläche (Deckfläche) nach der Integration in das Gerüst bzw. das Implantat auf der abluminalen Seite liegt, führen die anorganischen Verbindungen bei Kontakt mit Zellgewebe zu einer verstärkten Interaktion mit den Endothelzellen. Im Ergebnis kommt es zu einer schnelleren Inkorporation des Markerelements in die Gefäßwand.

Als organische Verbindung können Verbindungen ähnlich BMPs, die in der Orthopädie eine Beschleunigung der Osteosynthese bewirken, verwendet werden, welche ebenfalls die Inkorporation des Markerelements in die Gefäßwand unterstützen.

Die obige Aufgabenstellung wird außerdem durch ein Verfahren zur Herstellung eines Gerüsts mit einem Markerelement für ein Implantat aufweisend die obigen Schritte des Verfahrens zur Herstellung des Markerelements und den zusätzlichen Verfahrensschritt gelöst, bei dem der von dem Halbzeug abgetrennte, das Markerelement bildende Abschnitt in eine Öffnung (Eyelet) des Gerüsts eingebracht (eingesetzt) und in die Öffnung eingeklebt wird. Die Öffnung ist vorzugsweise durchgehend. Hierbei wird, wie oben bereits ausgeführt wurde, das Markerelement derart in das Gerüst eingeklebt, dass die erste Oberfläche des Abschnitts (Grundfläche des Markerelements), die durch den Beizvorgang entstanden ist, eine luminale Fläche und die zweite Oberfläche des Abschnitts (Deckfläche des Markerelements) mit der porösen Schicht eine abluminale Fläche an dem Gerüst bzw. dem Implantat bildet. Durch die luminale gebeizte Oberfläche des Markerelements wird die Gefahr der Bildung von Thromben reduziert, während durch die poröse Schicht auf der abluminalen Seite bzw. das Einwachsen des Markerelements in das Gewebe begünstigt und die Lokalelementbildung mit dem Gerüst verhindert wird.

In einem weiteren bevorzugten Ausführungsbeispiel ist das mindestens eine Markerelement mit einem Klebstoff, vorzugsweise mit einem polymerbasierten Klebstoff, an dem Gerüst befestigt. Es wurde nämlich erkannt, dass insbesondere durch eine stoffschlüssige Verbindung eine einfache und mechanisch den filigranen Gerüst nicht belastende Verbindung erzielt wird. Zusätzlich kann durch Anpassung der Form des Markerelements und der Form der Öffnung (Eyelet, Aufnahme) am Gerüst des Implantats, in welche das Markerelement eingebracht wird, ein Formschluss realisiert werden.

In einer bevorzugten Ausführungsform werden elastische polymere Klebstoffe verwendet. Geeignete elastische Klebstoffe umfassen Silikone, Polylactide, Polyhydroxybuttersäure, thermoplastische Elastomere (TPE) sowie deren Blends. Elastische Klebstoffe haben den Vorteil, dass sie zu einer verbesserten Trackability, also zur Anpassung an das umgebende Gewebe während des Vordringens der Endoprothese beim Einsetzen, beitragen, sodass ein vorzeitiger Verlust eines Markerelements vermieden wird.

Als vorteilhafter polymerbasierter Klebstoff kann beispielsweise Polyurethan oder ein degradierbares Polymer (z. B. PLLA wie L210 oder L214 von Evonik ) verwendet werden.

Diese Klebstoffe sind besonders bioverträglich und sind außerdem gut elektrisch isolierend.

Wie oben bereits erläutert wurde, ist es von Vorteil, wenn das mindestens eine Markerelement durch Kleben, vorzugsweise mittels eines oben erwähnten polymerbasierten Klebstoffs, an dem Gerüst, insbesondere in einer Öffnung des Gerüsts, befestigt wird, da das Festlegen des Markerelements an dem Gerüst einfach ist und automatisiert werden kann.

In einer Weiterbildung der Erfindung erfolgt das Einkleben mittels einer zuvor auf das von dem Halbzeug abgetrennte Markerelement aufgebrachten Polymerlösung, z.B. einer PLLA/CHCl₃-Lösung, wobei die Polymerlösung vorzugsweise mittels manuellem oder automatisiertem Eindrücken des Markerelements beispielsweise mit Hilfe eines Stempels oder einer Vakuumpinzette in die (ggf. leicht gehärtete) Polymerlösung und/oder Benetzen des Markerelements mit der Polymerlösung (z.B. mittels Aufsprühen) und/oder Auffüllen des Spalts zwischen Markerelement und gegenüber liegendem Rand der Öffnung des Gerüsts aufgebracht wird, und anschließendem Aushärten der Polymerlösung.

In einem weiteren Ausführungsbeispiel wird das Gerüst zumindest in einem vorgegebenen Bereich vor Einkleben des Markerelements mit einer Beschichtung enthaltend eine pharmazeutisch aktive Substanz (z.B. Immunsuppressiva, Zytostatika) versehen.

Hierbei wird unter einer "pharmazeutisch aktiven Substanz" (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom Menschen oder tierischen Körper erzeugte Wirkstoffe, wie Insulin, sowie zur Beseitigung oder Unschädlichmachen von Krankheitserregern, Tumoren, Krebszellen oder Körperfremdstoffen Einsatz findet. Als derartige Wirkstoffe kommen beispielsweise Everolimus, Sirolimus, Paclitaxel oder Heparin in Frage.

Alternativ oder zusätzlich wird das Gerüst zumindest in einem vorgegebenen Bereich nach dem Einkleben des mindestens einen Markerelements mit einer Beschichtung enthaltend eine pharmazeutisch aktive Substanz versehen.

Die obige Aufgabenstellung wird außerdem durch ein scheibenförmiges Markerelement für ein Implantat mit einer eine erste Oberfläche ausbildenden Grundfläche, einer eine zweite Oberfläche ausbildenden Deckfläche und mindestens einer die Grundfläche und die Deckfläche verbindenden Seitenfläche gelöst, wobei die Deckfläche nach Befestigung des Markerelements am Gerüst des Implantats eine abluminale Fläche bildet. Erfindungsgemäß ist die Deckfläche und gegebenenfalls auch mindestens eine Seitenfläche des Markerelements zumindest teilweise durch eine poröse Schicht abgedeckt, welche, wie oben bereits ausführlich beschrieben wurde, durch plasmaelektrolytische Behandlung erzeugt wurde.

In einem Ausführungsbeispiel weist die poröse Schicht eine Porengröße zwischen 0,1 µm und 2 µm und/oder eine Schichtdicke zwischen 0,3 µm und 10 µm, vorzugsweise zwischen 0,5 µm und 4 µm, auf.

Wie oben bereits ausgeführt wurde, weist das Markerelement in einem Ausführungsbeispiel eine riefenförmige Beizstruktur an der Grundfläche auf, wenn vor der plasmaelektrolytischen Behandlung eine Beizbehandlung des mindestens einen Abschnitts an dem Halbzeug mit einer Säure durchgeführt wurde.

Die obige Aufgabenstellung wird ferner gelöst durch ein Gerüst für ein Implantat mit einem oben angegebenen Markerelement, wobei das Markerelement in eine Öffnung (Eyelet) des Gerüsts derart eingeklebt ist, dass die Deckfläche des Markerelements eine abluminale Fläche bildet. Entsprechend kann die Grundfläche des Markerelements, welche die vorzugsweise riefenförmige Beizstruktur aufweist, eine abluminale Fläche ausbilden.

Die obige Aufgabenstellung wird mit den gleichen Vorteilen außerdem durch ein Implantat mit einem oben angegebenen Gerüst gelöst.

Die Erfindung wird nachfolgend anhand von Ausführungsbeispielen und unter Bezugnahme auf die Figuren erläutert. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbezügen.

Es zeigen schematisch:
- Fig. 1: einen Abschnitt eines Gerüsts eines Implantats in einer Ansicht von oben,
- Fig. 2: ein Markerelement in einer Ansicht von oben,
- Fig. 3a- 3b: Rasterelektronenmikroskopische Aufnahmen der Außenseite (Fig. 3a) und der Innenseite (Fig. 3b) eines Bereichs eines hohlzylindrischen Halbzeugs mit einer Vielzahl von Abschnitten für Markerelemente nach Entfernung der Schlackenreste und etwaiger Verunreinigungen in jeweils einer Ansicht von der jeweiligen Seite,
- Fig. 4: Innenseite des Bereichs des hohlzylindrischen Halbzeugs gemäß Fig. 3b mit einem Abschnitt für ein Markerelement nach dem Beizschritt in einer lichtmikroskopischen Aufnahme von der Seite,
- Fig. 5a -5c: Bereiche einer abluminalen Deckfläche eines Markerelements nach der plasmaelektrolytischen Behandlung in verschiedenen Vergrößerungen, wobei die Fig. 5b und 5c jeweils einen Kratzer in der Oberfläche entstanden durch mechanisches Eindrücken eines spitzen Gegenstands zeigen, in einer rasterelektronenmikroskopischen Aufnahme von oben und unterschiedlicher Vergrößerung sowie

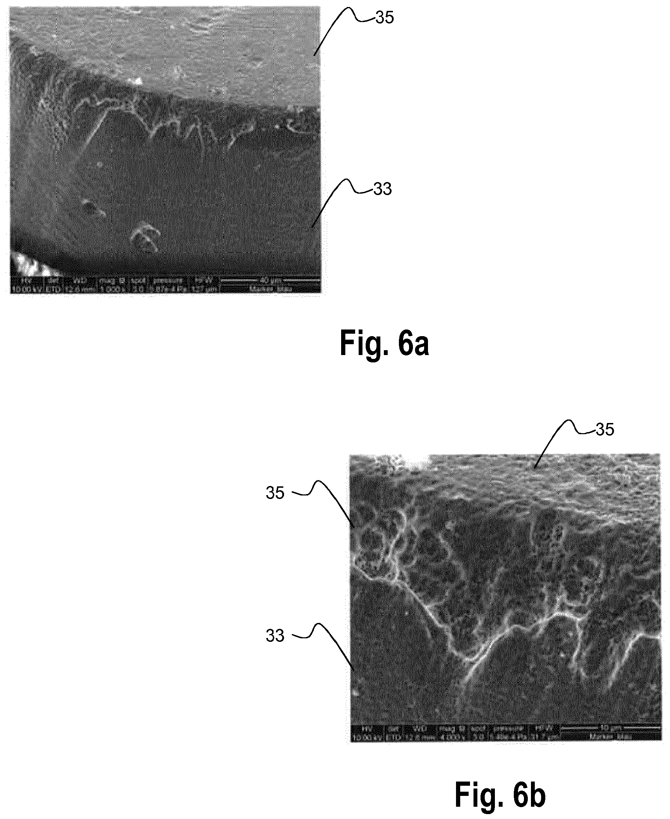

- Fig. 6a - 6b: einen Bereich der abluminalen Deckfläche und der Seitenfläche des Markerelements in einer schrägen rasterelektronenmikroskopischen Aufnahme von der Seite in unterschiedlicher Vergrößerung.

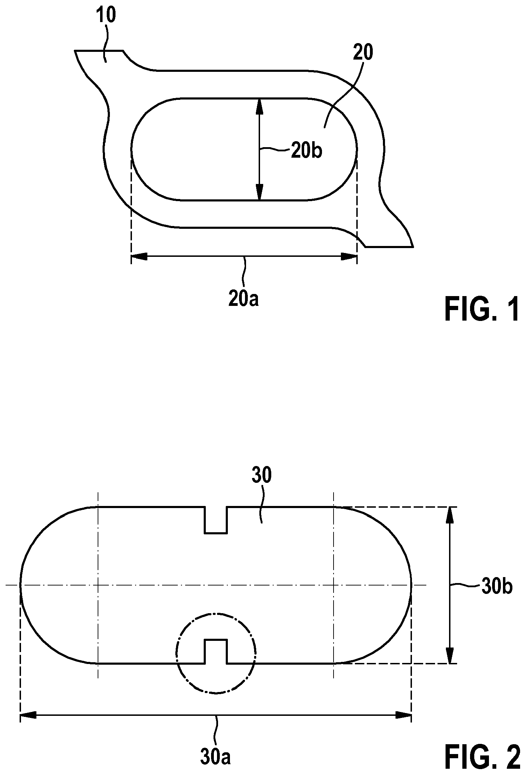

In Fig. 1 ist ein Abschnitt eines Gerüsts 10 eines erfindungsgemäßen Implantats in Form eines medizinischen Stents, beispielsweise bestehend aus der degradierbaren Magnesiumlegierung WE43, dargestellt. Das Bild zeigt eine beispielsweise am distalen oder proximalen Ende des Gerüsts 10 angeordnete durchgehende Öffnung (im Folgenden Eyelet) 20 mit einer abgerundeten Rechteckgrundform. Vorzugsweise sind am distalen und/oder am proximalen Ende des Gerüsts 10 des Implantats jeweils ein Eyelet 20 oder drei um 120° versetzte solche Eyelets 20 als Bestandteile des Gerüsts, beispielsweise an einer Strebe (Strut), vorgesehen. Hierbei ist das Gerüst vorzugsweise als hohlzylinderförmiges Gitter mit einer Vielzahl von Streben ausgebildet. Die Maße des Eyelets 20 betragen beispielsweise 800 µm (Abmessung 20a in Fig. 1) x 350 µm (Abmessung 20b in Fig. 1).

In dem Eyelet 20 kann ein röntgenopakes Markerelement 30 (siehe Fig. 2) angeordnet werden, das, wie nachfolgend beschrieben wird, mittels einer Kleberschicht an dem Eyelet 20 befestigt werden kann.

Das Markerelement 30 besteht beispielsweise überwiegend aus Tantal oder einer Tantallegierung. Die Dicke des Markerelements 30 beträgt beispielsweise 100 µm und entspricht vorzugsweise der Wanddicke des Gerüsts 10. Die Abmessungen des Markers betragen z.B. 750 µm (Abmessung 30a in Fig. 2) x 300 µm (Abmessung 30b in Fig. 2).

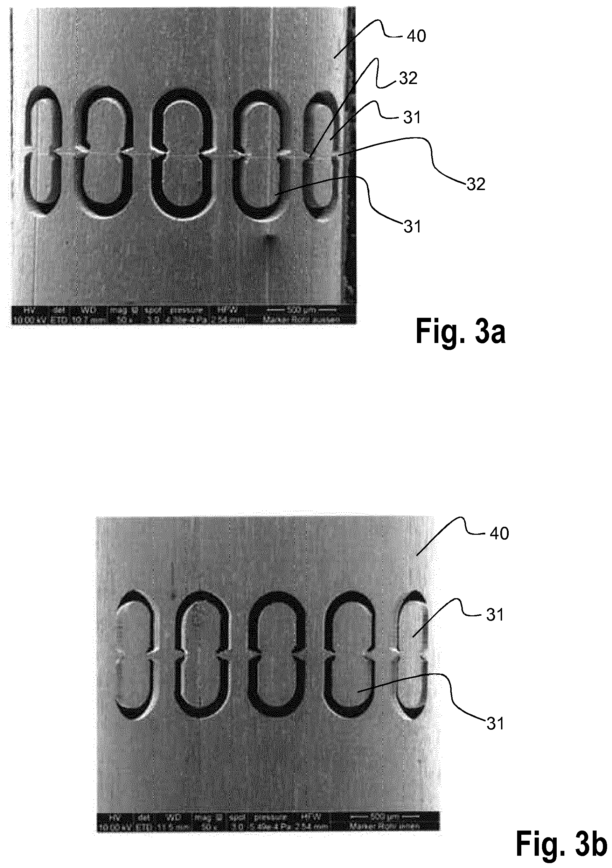

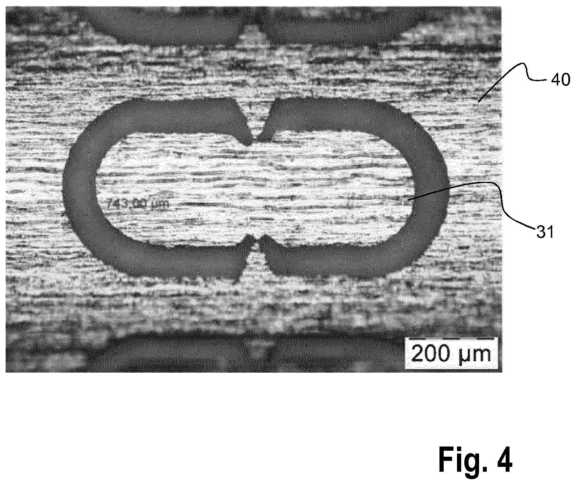

Zur Herstellung des Markerelements 30 wird zuerst mit Hilfe eines Lasers aus einem Halbzeug in Form eines gezogenen Hohlzylinders 40 aus Tantal oder einer Tantallegierung, beispielsweise mit einem Außendurchmesser von 2,5 mm, Abschnitte 31 mit der Form des Markerelements 30 herausgeschnitten. Hierbei ist jeder Abschnitt 31 noch über zwei Stege 32 mit dem Hohlzylinder 40 verbunden. Anschließend werden an dem Hohlzylinder 40 mit den Abschnitten 31 Schlackereste, Laserschneidgrate und etwaige Verunreinigungen durch Beizen mit einer Säuremischung aus z.B. HNO₃ und HF entfernt und danach einem mehrstufigen Spülschritt in VE-Wasser unterzogen. Für das Beizen wird der Hohlzylinder mit den Abschnitten 31 mehrfach in die Säuremischung eingetaucht. Die Beschaffenheit der Außenseite und die Innenseite eines solchen Hohlzylinders 40 mit den Abschnitten 31 nach diesem Schritt sind in Fig. 3a und b dargestellt. Fig. 4 zeigt die Innenseite des Hohlzylinders 40 mit einem Abschnitt 31 in einer vergrößerten Abbildung. Die Innenseite der Abschnitte 31, die auch als erste Oberfläche bezeichnet wird, (und somit die Grundfläche des hieraus hergestellten Markerelements 30) zeichnet sich durch eine durch den Ziehvorgang des Hohlzylinders 40 und das anschließende Beizen erzeugte Struktur mit einer Vielzahl von Riefen bzw. Rillen aus. Diese verlaufen nach der Anordnung des Markerelements 30 in das Gerüst 10 parallel zur Achse des Gerüsts bzw. parallel zur Fließrichtung des durch den Stent fließenden Blutes. Die Struktur wirkt einer Adhäsion der Blutplättchen entgegen und senkt somit das Thromboserisiko, auch und vor allem in der Zeit nach der Auflösung einer optionalen, auf der Oberfläche des Gerüsts aufgebrachten PLLA-Schicht.

Anschließend wird ein Ballon mit einem Durchmesser von 2,35 mm (nach Expansion des Ballons) in den Hohlzylinder 40 eingebracht. Die Expansion des Ballons erfolgt mittels Druckbeaufschlagung. Nach der Aufweitung liegt der Ballon formschlüssig an der Innenseite des Hohlzylinders 40 an und dichtet diese vollständig ab. So ist die außen liegende zweite Oberfläche der Abschnitte 31 des Hohlzylinders 40 und die Seitenflächen 33 weiteren Prozessschritten zugänglich, ohne dass die in Fig. 4 gezeigte erste Oberfläche der Abschnitte 31 des Hohlzylinders mit weiteren Medien in Kontakt tritt.

Der Hohlzylinder mit den Abschnitten 31 wird nun einer plasmaelektrolytischen Behandlung unterzogen. Die Oxidation des chemisch stabilen Elements Tantals wird durch die, bei Badspannungen >180 V entstehenden, lokal begrenzten Plasmaentladungen hervorgerufen. Dabei rastern einzelne Plasmaentladungen die nicht abgedeckte Oberfläche der Abschnitte 31 systematisch ab, nämlich die äußere Oberfläche (siehe Fig. 3a) und zumindest teilweise die durch das Laserschneiden frei liegenden Seitenoberflächen 33 der Abschnitte 31.

Anschließend wird der plasmaelektrolytisch behandelte Hohlzylinder 40 mit den Abschnitten 31mehrfach in VE-Wasser gespült und die Abschnitte 31 werden mittels Durchtrennen der Stege 32 (Herausbrechen) von dem Hohlzylinder 40 abgetrennt. Jeder abgetrennte Abschnitt bildet ein Markerelement 30 aus. Das Spülen und Ausbrechen der Abschnitt 31 erfolgt hierbei nach dem Entfernen des die Innenseite abdeckenden Ballons aus dem Hohlzylinder 40.

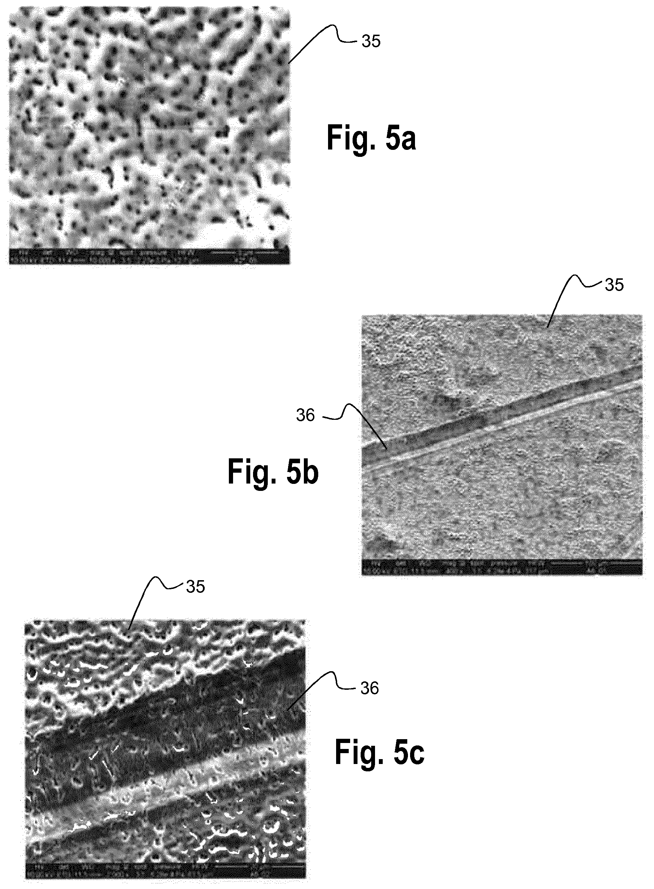

Das Markerelement 30 erhält durch die plasmaelektrolytische Behandlung eine verfahrenstypische poröse Oberfläche, die zum größten Teil aus Ta₂O₅ besteht. Die Dicke der durch die plasmalelektrolytische Behandlung erzeugten porösen Schicht 35 beträgt beispielsweise zwischen 0,5 µm und 4 µm. Aufgrund des Konversionscharakters der Plasmaentladungen bleibt die ursprüngliche Außengeometrie des Markerelements 30 gegenüber dem Abschnitt 31 erhalten. Die durch die plasmaelektrolytische Behandlung erzeugte poröse Schicht 35 ist in den Fig. 5a bis 5c gezeigt. Die poröse Schicht 35 weist aufgrund der stofflichen Verzahnung mit dem darunter liegenden metallischen Substrat aus Tantal oder einer Tantallegierung eine hohe Haftfestigkeit auf. Dies wird anhand der in Fig. 5b und 5c gezeigten Kratzer 36 deutlich, die durch Eindrücken mit einem spitzen Gegenstand (Indenter) erzeugt sind. Die Porenstruktur der porösen Schicht 35 bewirkt ein Stopp von Rissen, die durch die mechanische Einwirkung erzeugt werden.

Die Oberflächenstrukturierung durch die Schicht 35 führt auch zu einer wesentlichen Vergrößerung der realen Oberfläche des Markerelements 30 mindestens um den Faktor 2. Dies ist eine wesentliche Voraussetzung für eine hohe Immobilisierungskapazität des Markerelements 30.

Für die Anordnung an einem Gerüst 10 erfolgt die Entnahme des Hohlzylinders 40 aus dem Exsikkator, Einbau in ein Entnahmegestell und unverzügliches mechanisches Greifen und Herausbrechen eines Abschnittes 31 aus dem Hohlzylinder, so dass das Markerelement 30 entsteht. Das Gerüst 10 wurde kurz zuvor mit einer Polymerwirkstoffbeschichtung (z.B. PLLA/Sirolimus) versehen. Nach der Positionierung des Markerelements 30 im Eyelet 20 wird dieses in die noch plastisch verformbare, lösemittelhaltige Beschichtung (z. B. PLLA in Chloroform 2/98) gedrückt. Zusätzlich kann der entstandene Spalt zwischen Markerelement 30 und Gerüst 10 mit der Polymerlösung aufgefüllt werden. Eine ausreichende Haltekraft des Markerelements 30 im Eyelet 20 wird danach durch das Abdampfen des Lösemittels aus der Lösung und damit einem Aushärten der Polymerbeschichtung bewirkt. Dadurch wird erreicht, dass sowohl die biofunktionalisierte, abluminale als auch die in Blutflussrichtung strukturierte, luminale Markerseite nicht mit einer Polymerschicht bedeckt ist. Anschließend kann die Montage des Gerüsts 10 mit dem Markerelement 30 in das Kathetersystem erfolgen.

Durch die poröse Schicht 35, die durch die plasmaelektrolytische Behandlung gebildet wird, wird sichergestellt, dass kein Metall-/Metallkontakt zwischen Gerüst 10 und Material des Markerelements 30 entsteht, dadurch die Lokalelementbildung sicher unterbunden wird und keine Beeinflussung des weiteren Degradationsverlaufs des Gerüsts 10 stattfindet.

In einem weiteren alternativen Ausführungsbeispiel kann das Markerelement 30 vor der Positionierung in dem Eyelet 20 in eine Polymerlösung (z. B. PLLA in Chloroform 2/98) getaucht werden. Ein Ausfüllen des Spalts zwischen Markerelement 30 und Gerüst 10 ist bei dieser Ausführungsform nicht unbedingt erforderlich. Nach der Positionierung des Markerelements 30 im Eyelet 20 wird eine ausreichende Haltekraft des Markerelements 30 im Eyelet 20 durch das Aushärten der Polymerbeschichtung bewirkt. Danach kann die Beschichtung des Gerüsts 10 einschließlich Markerelement 30 mit einer Polymerwirkstoffschicht und anschließend die Montage des Gerüsts in das Kathetersystem erfolgen.

In einem alternativen Ausführungsbeispiel kann der plasmaelektrolytisch behandelte Hohlzylinder 40 nach dem Entfernen des Ballons mehrfach in VE-Wasser gespült und danach zusätzlich in eine Lösung getaucht werden, die den Crosslinker z.B. APTES (Aminopropyltriethoxysilan) enthält. Durch Anlegen eines Unterdrucks in einem Exsikkator saugen sich die Poren mit der Lösung voll. Hierbei kann die Reaktion auf der Oberfläche der Abschnitte 31 auch thermisch aktiviert werden. Anschließend erfolgt zusätzlich eine Reaktion mit Adhäsionspeptiden (z.B. RGD-Peptiden), die eine besondere Affinität zu den Zellen der Gefäßwand aufweisen. Diese binden kovalent an die mit APTES aktivierte Oberfläche. Danach erfolgt eine Spülung/Trocknung des Hohlzylinders 40 mit den Abschnitten 31 und Ablage in einem Exsikkator. Während der vaskulären Intervention des Gerüsts 10 kommt auch die Umgebung des Markerelements 30 in Kontakt mit Blut. Dieses Ausführungsbeispiel hat daher den Vorteil, dass die Poren der abluminalen Deckfläche des Markerelements 30 mit der porösen Schicht 35 aufgrund der mittels APTES angebundenen Adhäsionspeptide Blutbestandteile aufnehmen, die zu einer verbesserten Anhaftung und Einheilung führen. So wird sichergestellt, dass der Marker schnell einwächst und selbst nach erfolgter Degradation des Gerüsts 10 und der Polymerwirkstoffschicht sicher eingewachsen im Gefäßgewebe verbleibt.

In einem weiteren alternativen Ausführungsbeispiel wird nach der plasmaelektrolytischen Behandlung der Hohlzylinder 40 mit den Abschnitten 31 nach Entfernen des Ballons mehrfach in VE- Wasser gespült und danach zusätzlich in eine Lösung getaucht, die den Crosslinker APTES (Aminopropyltriethoxysilan) enthält. Durch Anlegen eines Unterdrucks in einem Exsikkator saugen sich die Poren mit der Lösung voll. Hierbei kann die Reaktion auf der Markeroberfläche auch thermisch aktiviert werden. Anschließend erfolgt zusätzlich eine Reaktion mit Proteinen, wie Wachstumsfaktoren (z.B. VEGF) oder Antikörpern, die eine besondere Affinität zu den Zellen der Gefäßwand aufweisen. Diese binden kovalent an die mit APTES aktivierte Oberfläche. Danach erfolgt eine Spülung/Trocknung des Hohlzylinders 40 und Ablage in einem Exsikkator. Auch bei diesem Ausführungsbeispiel wird durch die immobilisierten Proteine bewirkt, dass das Markerelement 30 schnell in das Gefäßgewebe einwächst und selbst nach erfolgter Degradation des Gerüsts 10 und der auf diesem angeordneten Polymerwirkstoffschicht im Gewebe verbleibt.

## Patentansprüche

1. Verfahren zur Herstellung eines Markerelements (30) für ein Implantat aus einem plattenförmigen oder hohlkörperförmigen Halbzeug (40) bestehend aus dem Material des Markerelements (30) aufweisend die folgenden Schritte:
- Ausschneiden mindestens eines Abschnitts (31) aus dem Halbzeug derart, dass der Abschnitt über mindestens einen Steg (32) mit dem übrigen Material des Halbzeugs (40) verbunden ist,
- während der Abschnitt (31) weiter über den mindestens einen Steg (32) mit dem Halbzeug verbunden ist:
- Abdecken einer ersten Oberfläche des Abschnitts (31) und
- anschließende plasmaelektrolytische Behandlung mindestens einer zweiten Oberfläche des Abschnitts (31), wobei die mindestens eine zweite Oberfläche von der ersten Oberfläche verschieden ist, und der Seitenfläche des Markerelements, die quer zur zweiten Oberfläche verläuft, wobei durch die plasmaelektrolytische Behandlung eine poröse Schicht (35) zumindest an der zweiten Oberfläche des Abschnitts (31) und zumindest teilweise an der Seitenfläche ausgebildet wird, sowie
- Abtrennen des Abschnitts (31) mittels Durchtrennen des mindestens einen Stegs (32), wobei der abgetrennte Abschnitt des Halbzeugs das Markerelement (30) bildet.

2. Verfahren nach Anspruch 1, **dadurch gekennzeichnet, dass** die plasmaelektrolytische Behandlung bei einer maximalen Badspannung von über 180 V mit einer gepulsten Spannungsquelle, die mit dem Halbzeug (40) elektrisch leitend verbunden ist, durchgeführt wird.

3. Verfahren nach einem der vorhergehenden Ansprüche, **dadurch gekennzeichnet, dass** durch die plasmaelektrolytische Behandlung die poröse Schicht (35) auch zumindest teilweise an einer quer zur zweiten Oberfläche verlaufenden Seitenfläche (33) des ausgeschnittenen Abschnitts (31) gebildet wird.

4. Verfahren nach einem der vorhergehenden Ansprüche, **dadurch gekennzeichnet, dass** das Halbzeug (30) im Bereich des mindestens einen ausgeschnittenen Abschnitts (31) vor dem Abdecken der ersten Oberfläche des Abschnitts (31) einer Beizbehandlung mit einer Säure unterzogen wird.

5. Verfahren nach einem der vorhergehenden Ansprüche, **dadurch gekennzeichnet, dass** das Halbzeug im Bereich des mindestens einen ausgeschnittenen Abschnitts (31) nach der plasmaelektrolytischen Behandlung zuerst in eine Lösung aufweisend einen Crosslinker und anschließend in eine Mischung aufweisend mindestens ein Material aus der Gruppe enthaltend Proteine, weitere Wachstumsfaktoren, Enzyme, Antikörper und Peptide getaucht wird.

6. Verfahren nach einem der Ansprüche 1 bis 3, **dadurch gekennzeichnet, dass** eine elutionsfähige organische Verbindung oder eine anorganische Verbindung in der Porenstruktur der porösen Schicht immobilisiert wird.

7. Verfahren zur Herstellung eines Gerüsts (10) mit einem Markerelement (30) für ein Implantat aufweisend die Schritte des Verfahrens zur Herstellung des Markerelements (30) nach einem der vorhergehenden Ansprüche und den zusätzlichen Verfahrensschritt, dass der von dem Halbzeug (40) abgetrennte, das Markerelement (30) bildende Abschnitt (31) in eine Öffnung (20) des Gerüsts (10) eingebracht und in die Öffnung (20) eingeklebt wird.

8. Verfahren nach Anspruch 7, **dadurch gekennzeichnet, dass** das Einkleben mittels einer zuvor auf das von dem Halbzeug (40) abgetrennte Markerelement (30) aufgebrachte Polymerlösung, wobei die Polymerlösung mittels Eindrücken und/oder Auffüllen des Spalts zwischen Markerelement und gegenüber liegendem Rand der Öffnung (20) des Gerüsts (10) aufgebracht wird, und Aushärten der Polymerlösung erfolgt.

9. Verfahren nach einem der Ansprüche 7 bis 8, **dadurch gekennzeichnet, dass** das Gerüst (10) zumindest in einem vorgegebenen Bereich vor Einkleben des Markerelements (30) mit einer Beschichtung enthaltend eine pharmazeutisch aktive Substanz versehen wird.

10. Verfahren nach einem der Ansprüche 7 bis 8, **dadurch gekennzeichnet, dass** das Gerüst (10) zumindest in einem vorgegebenen Bereich nach dem Einkleben des Markerelements (30) mit einer Beschichtung enthaltend eine pharmazeutisch aktive Substanz versehen wird.

11. Scheibenförmiges Markerelement für ein Implantat mit einer eine erste Oberfläche ausbildenden Grundfläche, einer eine zweite Oberfläche ausbildenden Deckfläche und mindestens einer die Grundfläche und die Deckfläche verbindenden Seitenfläche (33), wobei die Deckfläche nach Befestigung des Markerelements (30) am Gerüst (10) des Implantats eine abluminale Fläche bildet, **dadurch gekennzeichnet, dass** die Deckfläche und Seitenfläche (33) des Markerelements (30) zumindest teilweise durch eine poröse Schicht (35) abgedeckt ist, welche durch plasmaelektrolytische Behandlung erzeugt ist, während die Grundfläche keine Veränderung durch die plasmaelektrolytische Behandlung aufweist.

12. Markerelement (30) nach Anspruch 11, **dadurch gekennzeichnet, dass** die poröse Schicht (35) einen mittleren Porendurchmesser zwischen 0,1 µm und 2 µm und/oder eine Schichtdicke zwischen 0,3 µm und 10 µm, vorzugsweise zwischen 0,5 µm und 4 µm, aufweist.

13. Markerelement (30) nach einem der Ansprüche 11 bis 12, **dadurch gekennzeichnet, dass** das Markerelement (30) eine riefenförmige Beizstruktur an der Grundfläche aufweist.

14. Gerüst (10) für ein Implantat mit einem Markerelement (30) nach einem der Ansprüche 11 bis 13, **dadurch gekennzeichnet, dass** das Markerelement (30) in eine Öffnung (20) des Gerüsts (10) derart eingeklebt ist, dass die Deckfläche des Markerelements (30) eine abluminale Fläche bildet.

15. Implantat mit einem Gerüst (10) nach Anspruch 14.

## Claims

1. A method for producing a marker element (30) for an implant from a planar or hollow body-shaped semi-finished product (40) consisting of the material of the marker element (30), comprising the following steps:
- cutting out at least one portion (31) from the semi-finished product, in such a way that the portion is connected via at least one web (32) to the rest of the material of the semi-finished product (40),
- whilst the portion (31) is still connected to the semi-finished product via the at least one web (32):
- covering a first surface of the portion (31) and
- performing a subsequent plasma electrolytic treatment of at least a second surface of the portion (31), wherein the at least one second surface is different from the first surface, and the side face of the marker element, which runs transversely to the second surface, wherein a porous layer (35) is formed at least on the second surface of the portion (31) and at least partially on the side face by the plasma electrolytic treatment, and
- separating the portion (31) by severing the at least one web (32), wherein the separated portion of the semi-finished product forms the marker element (30).

2. The method according to claim 1, **characterised in that** the plasma electrolytic treatment is performed at a maximum bath voltage of more than 180 V with a pulsed voltage source which is electrically conductively connected to the semi-finished product (40).

3. The method according to either one of the preceding claims, **characterised in that** the porous layer (35) is also formed at least in part on a side face (33) of the cut-out portion (31) transverse to the second surface by means of the plasma electrolytic treatment.

4. The method according to any one of the preceding claims, **characterised in that** the semi-finished product (30), in the region of the at least one cut-out portion (31), is subjected to a pickling treatment with an acid prior to the covering of the first surface of the portion (31).

5. The method according to any one of the preceding claims, **characterised in that** the semi-finished product, in the region of the at least one cut-out portion (31), after the plasma electrolytic treatment is firstly immersed in a solution comprising a crosslinker and is then immersed in a mixture comprising at least one material from the group containing proteins, further growth factors, enzymes, antibodies and peptides.

6. The method according to any one of clams 1 to 3, **characterised in that** an organic compound or an inorganic compound capable of elution is immobilised in the pore structure of the porous layer.

7. A method for producing a framework (10) comprising a marker element (30) for an implant, comprising the steps of the method for producing the marker element (30) according to any one of the preceding claims and the additional method step that the portion (31) separated from the semi-finished product (40) and forming the marker element (30) is introduced into an opening (20) of the framework (10) and is glued into the opening (20).

8. The method according to claim 7, **characterised in that** the gluing is realised by means of a polymer solution being applied beforehand to the marker element (30) separated from the semi-finished product (40), wherein the polymer solution is applied by being pressed in and/or filling the gap between the marker element and opposite edge of the opening (20) of the framework (10), and by means of the polymer solution being cured.

9. The method according to any one of clams 7 to 8, **characterised in that** the framework (10) is provided, at least in a predefined region, with a coating containing a pharmaceutically active substance, prior to the gluing of the marker element (30).

10. The method according to any one of claims 7 to 8, **characterised in that** the framework (10) is provided, at least in a predefined region, with a coating containing a pharmaceutically active substance, after the gluing of the marker element (30).

11. A disc-shaped marker element for an implant having a bottom face forming a first surface, a top face forming a second surface, and at least one side face (33) connecting the bottom face and the top face, wherein the top face forms an abluminal face once the marker element (30) has been secured to the framework (10) of the implant, **characterised in that** the top face and side face (33) of the marker element (30) is covered at least in part by a porous layer (35), which was generated by plasma electrolytic treatment, while the bottom face has no change as a result of the plasma electrolytic treatment.

12. The marker element (30) according to claim 11, **characterised in that** the porous layer (35) has a mean pore diameter between 0.1 µm and 2 µm and/or a layer thickness between 0.3 µm and 10 µm, preferably between 0.5 µm and 4 µm.

13. The marker element (30) according to any one of claims 11 to 12, **characterised in that** the marker element (30) has a groove-shaped pickling structure on the bottom face.

14. A framework (10) for an implant comprising a marker element (30) according to any one of clams 11 to 13, **characterised in that** the marker element (30) is glued into an opening (20) of the framework (10) in such a way that the top face of the marker element (30) forms an abluminal face.

15. An implant with a framework (10) according to claim 14.

## Revendications

1. Procédé de fabrication d'un élément marqueur (30) pour un implant à base d'un produit semi-fini (40) en forme de plaque ou en forme de corps creux constitué d'un matériau de l'élément marqueur (30) présentant les étapes suivantes :
- découpage d'au moins un segment (31) du produit semi-fini de telle façon que le segment soit relié avec le matériau restant du produit semi-fini (40) par le biais d'au moins un pontage (32),
- pendant que le segment (31) reste relié avec le produit semi-fini par le biais de l'au moins un pontage (32) :
- recouvrement d'une première surface du segment (31) et
- traitement électrolytique par plasma ultérieur d'au moins une deuxième surface du segment (31), où l'au moins une deuxième surface est différente de la première surface, et de la surface latérale de l'élément marqueur qui s'étend perpendiculairement par rapport à la deuxième surface, où une couche poreuse (35) se forme par le traitement électrolytique par plasma au moins sur la deuxième surface du segment (31) et au moins partiellement sur la surface latérale, ainsi que
- séparation du segment (31) au moyen d'une séparation de l'au moins un pontage (32), où le segment séparé du produit semi-fini forme l'élément marqueur (30).

2. Procédé selon la revendication 1, **caractérisé en ce que** le traitement électrolytique par plasma est effectué à une tension de bain maximale de plus de 180 V avec une source de tension pulsée qui est reliée de manière conductrice électriquement avec le produit semi-fini (40).

3. Procédé selon l'une des revendications précédentes, **caractérisé en ce que** la couche poreuse (35) est également formée au moins partiellement sur une surface latérale (33) du segment (31) découpé s'étendant perpendiculairement par rapport à la deuxième surface par le traitement électrolytique par plasma.

4. Procédé selon l'une des revendications précédentes, **caractérisé en ce que** le produit semi-fini (30), dans la zone de l'au moins un segment (31) découpé, est soumis à un traitement de gravure avec un acide avant le recouvrement de la première surface du segment (31).

5. Procédé selon l'une des revendications précédentes, **caractérisé en ce que** le produit semi-fini, dans la zone de l'au moins un segment (31) découpé, est immergé, après le traitement électrolytique par plasma, d'abord dans une solution présentant un agent de réticulation et ensuite dans un mélange présentant au moins un matériau du groupe contenant des protéines, d'autres facteurs de croissance, des enzymes, des anticorps et des peptides.

6. Procédé selon l'une des revendications 1 à 3, **caractérisé en ce qu'**un composé organique ou un composé inorganique pouvant être élué est immobilisé dans la structure poreuse de la couche poreuse.

7. Procédé de fabrication d'un échafaudage (10) avec un élément marqueur (30) pour un implant présentant les étapes du procédé de fabrication de l'élément marqueur (30) selon l'une des revendications précédentes et l'étape de procédé complémentaire que le segment (31) séparé du produit semi-fini (40) formant l'élément marqueur (30) est introduit dans un orifice (20) de l'échafaudage (10) et est collé dans l'orifice (20).

8. Procédé selon la revendication 7, **caractérisé en ce que** le collage a lieu au moyen d'une solution de polymère rapportée auparavant sur l'élément marqueur (30) séparé du produit semi-fini (40), où la solution de polymère est rapportée au moyen d'un pressage et/ou d'un remplissage de la fente entre l'élément marqueur et le bord de l'orifice (20) de l'échafaudage (10) lui faisant face, et d'un durcissement de la solution de polymère.

9. Procédé selon l'une des revendications 7 à 8, **caractérisé en ce que** l'échafaudage (10) est muni au moins sur une zone prédéfinie d'un revêtement contenant une substance pharmaceutiquement active avant le collage de l'élément marqueur (30).

10. Procédé selon l'une des revendications 7 à 8, **caractérisé en ce que** l'échafaudage (10) est muni au moins sur une zone prédéfinie d'un revêtement contenant une substance pharmaceutiquement active après le collage de l'élément marqueur (30).

11. Elément marqueur en forme de disque pour un implant avec une surface de base formant une première surface, une surface de recouvrement formant une deuxième surface et au moins une surface latérale (33) reliant la surface de base et la surface de recouvrement, où la surface de recouvrement forme une surface abluminale après la fixation de l'élément marqueur (30) sur l'échafaudage (10) de l'implant, **caractérisé en ce que** la surface de recouvrement et la surface latérale (33) de l'élément marqueur (30) sont au moins partiellement recouvertes d'une couche poreuse (35), laquelle est créée par un traitement électrolytique par plasma tandis que la surface de base ne présente aucune modification par le traitement électrolytique par plasma.

12. Elément marqueur (30) selon la revendication 11, **caractérisé en ce que** la couche poreuse (35) présente un diamètre de pores moyen entre 0,1 µm et 2 µm, et/ou une épaisseur de couche entre 0,3 µm et 10 µm, de préférence, entre 0,5 µm et 4 µm.

13. Elément marqueur (30) selon l'une des revendications 11 à 12, **caractérisé en ce que** l'élément marqueur (30) présente une structure gravée en forme de rayures sur la surface de base.

14. Echafaudage (10) pour un implant avec un élément marqueur (30) selon l'une des revendications 11 à 13, **caractérisé en ce que** l'élément marqueur (30) est collé dans un orifice (20) de l'échafaudage (10) de telle manière que la surface de recouvrement de l'élément marqueur (30) forme une surface abluminale.

15. Implant pourvu d'un échafaudage (10) selon la revendication 14.
